# EUROPEAN PATENT APPLICATION

(11) **EP 2 000 532 A1**
(43) Date of publication of application: **10.12.2008**
(21) Application number: 08105435.5
(22) Date of filing: 01.08.2000
(51) Int. Cl.: C12N 15/02, C12N 15/06, C12N 15/08, C07K 16/00, C12P 21/08, G01N 33/53, A61K 39/00, C07K 16/40, C12N 9/02

(54) **Methods of measuring human cytochrome P450 (CYP2C8/9)**

(30) Priority: 05.08.1999 US 147315 P; 13.09.1999 US 153638 P
(62) Divisional of application: 00955297.7
(71) Applicant: Merck & Co., Inc., Rahway, NJ 07065-0907 (US)
(72) Inventor: Mei, Qin, Rahway, NJ 07065-0907 (US); Rushmore, Thomas H., Rahway, NJ 07065-0907 (US); Shou, Magang, Rahway, NJ 07065-0907 (US)
(74) Representative: Horgan, James Michael Frederic

(57) **Abstract**

Monoclonal antibodies (MAbs) have been developed to human cytochrome P450 2C8/9 (CYP2C8/9) by the fusion of myeloma tumour cells with isolated B-lymphocytes from spleens of mice immunised with baculovirus-expressed human CYP2C9; the MAbs for CYP2C9 are very powerful inhibitors of CYP2C8/9 activity and stray binders of the enzymes on Western blot and exhibit no cross-reactivity towards any of the other P450 subfamily members; based on these features the MAbs are useful tools for measuring CYP2C8/9 and assessing the role of CY2C8/9 in the metabolism of various compounds.

## Description

### FIELD OF THE INVENTION

The present invention relates to monoclonal antibodies (MAbs) specific for human cytochrome P450 3A4 (CYP3A4) and human cytochrome P450 2C9, assays using said antibodies and hybridomas producing specific antibodies.

### BACKGROUND OF THE INVENTION

Although the precise number of cytochrome P450 isoforms is unknown, there are at least fifteen forms of basal and inducible P450s derived from P450 families 1, 2, and 3, which play a role in the metabolism of most xenobiotics. The P450s identified so far are represented by multiple forms, and some exist at very low concentrations in tissues. Their substrate and product specificity often overlap. In addition, the levels of many P450s change in response to environmental, hormonal or nutritional influences, or are determined by genetic polymorphism. These factors have greatly limited our understanding of the precise role of individual cytochrome P450s in the metabolism, activation and detoxification of different cytochrome P450 substrates and, further, have prevented our understanding of the relationship between P450 phenotype in various tissues and an individual's responsiveness and sensitivity to drugs.

Developing reagent(s) such as inhibitory monoclonal antibodies that could precisely define a role of a single P450 molecule or even a class of P450 molecules in the metabolism of a particular drug would be of tremendous benefit to the field of drug metabolism. Monoclonal antibodies (MAbs) are ideally suited for these types of inquiries (i.e., the detailed analysis of specific molecules, such as the cytochrome P450 molecules above) in that they are chemically pure reagents that recognize a single antigenic determinant or epitope on the surface of a P450 molecule resulting in the inhibition of the catalytic activity associated with same. The measurement of P450 inhibition in different tissue preparations, thus, allows one to estimate the contribution of a particular P450 to the metabolism of various compounds in the tissue preparations, e.g., in human liver microsomes containing multiple P450s.

Towards this end, a hybridoma clone producing MAbs (MAb-A334™, IgM) that inhibit the enzymatic activity of cytochrome P450 3A4 (CYP3A4) probed with various substrates was developed (Gelboin et al., 1995 Biochem. Pharmacol. 50(11):1841-1850). The MAbs, however, displayed only partial inhibition (45∼90%) of most recombinant CYP3A4-mediated reactions (up to 100 µl ascites/mL incubation), and the inhibitory cross-reactivity of the MAb with other subfamilies of P450 was not fully investigated by using individual recombinant P450s, or P450- marker assays with human liver microsomes. Further, the MAb exhibited no immunoblot activity for CYP3A4 antigen on a Western blot impairing the use of same to characterize the quantitative role of CYP3A4 in the metabolism of a given drug. Instead, Gelboin proposes a supplementing monoclonal antibody, MAb 275-1-2, which, while lacking any inhibitory activity against CYP3A4, provides a Western blot against CYP3A4.

CYP3A4 remains of considerable interest and represents one of the best-studied members of the human P450 superfamily. Interest has focused on this P450 due to its abundance in human liver, inducibility by numerous agents (e.g., glucocorticoids, phenobarbital, etc.), and because of its prominent role in the metabolism of a large number of clinically relevant drugs.

A monoclonal antibody specific for CYP3A4, which both provides an immunoblot reaction against CYP3A4 and effectively inhibits CYP3A4-mediated reactions would contribute greatly towards the phenotyping of CYP3A4 in preclinical and clinical drug metabolism. Such a MAb could be ideally used in assays to assess the contribution of a particular P450 to the metabolism of a suspect drug in human tissue preparation. Further, said MAb could be used to determine the expression level of CYP3A4 in various tissues by a variety of immunoassays, including ELISA, western blot and immunohistochemical methods.

Another cytochrome of particular interest is cytochrome P450 2C9 (CYP2C9). CYP2C9 is a major human hepatic cytochrome P450 which plays a principal role in the oxidation of (S)-warfarin, tolbutamide, tienilic acid, losartan, diazepam, diclofenac, ibuprofen, nifedipine, mephenytoin, flurbiprofen, phenytoin, progesterone, testosterone and other drugs. Interest has focused on this P450 isoform due to its abundance in human liver, inducibility by numerous agents including rafampin and phenobarbital, and its prominent role in the metabolism of a large number of clinically relevant drugs. Phenotyping of CYP2C9 in preclinical and clinical drug metabolism currently represents one of the major goals toward generating a full understanding of the pharmacological, toxicological and pharmacokinetic consequences of drug metabolism. An inhibitory monoclonal antibody to CYP2C9 has not been developed. A monoclonal antibody that is a powerful inhibitor of CYP2C9- catalyzed reactions and a strong binder on Westernblot to the CYP2C9 antigen would be desired, particularly one that exhibits no cross-reaction towards any other P450 subfamily members. Such an antibody would be invaluable for use as a precise and sensitive probe for functional analysis of CYP2C8/9 in studies of human drug metabolism.

### SUMMARY OF THE INVENTION

The present invention relates to monoclonal antibodies specific for human cytochrome P450s 3A4 and 2C9.

The present invention further relates to a monoclonal antibody specific for human cytochrome P450 3A4 which was developed by the fusion of myeloma tumor cells with isolated B-lymphocytes from spleens of mice immunized with baculovirus-expressed human CYP3A4.

The present invention further relates to monoclonal antibodies specific for human CYP3A4 of the isotype IgG₂ₐ wherein the antibody exhibits cross-reactivity with human CYP3A5, human fetal CYP3A7 and Rhesus monkey testosterone 6β-hydroxylase.

A preferred embodiment of the present invention is a monoclonal antibody specific for human CYP3A4 of the isotype IgG₂ₐ which exhibits cross-reactivity with human CYP3A5, human fetal CYP3A7 and monkey testosterone 6β-hydroxylase but does not cross-react with human CYP1A1, CYP1A2, CYP2A6, CYP2B6, CYP2C8, CYP2C9, CYP2C19, CYP2D6, CYP2E1 or testosterone 6β-hydroxylase in dog, rat and mouse liver microsomes. An antibody in accordance with this description is MAb 10-1-1 as illustrated in the examples. This antibody was deposited with the American Type Culture Collection (ATCC), 10801 University Boulevard, Manassas, VA 20110-2209, in accordance with the Budapest Treaty on July 14, 1999 and assigned an accession number of PTA-351.

Another embodiment of the present invention relates to antibodies as described above wherein the antibody inhibits at least about 90% of human CYP3A4 activity and, preferably, at least about 95% of the activity. Notably, only 1 and 5 µl (0.5 µM and 2.5 µM of IgG₂ₐ) of the MAb mouse ascites in one mL incubation containing 20 pmol of CYP3A4 inhibited strongly the testosterone 6β-hydroxylation by 95% and 99%, respectively, rendering the MAb described herein a potent inhibitor of CYP3A4-catalyzed reactions. Inhibition of human CYP3A4 activity is measured by the inhibition of CYP3A4-catalyzed reactions such as testosterone 6β-hydroxylation, diazepam 3-hydroxylation and N-demethylation. Reactions catalyzed by CYP3A4 are known in the art as are methods by which to measure the resultant degree of metabolism. One way by which the degree of metabolism could be measured is by measuring the substrate and product levels after stopping the reaction and comparing said levels with a control sample wherein antibody was not added. For example, the reaction could be stopped by the addition of dichloromethane, the metabolites then extracted from the aqueous phase to the organic phase, the organic phase dried out, and the residue of the sample applied to a High Performance Liquid Chromatograph (HPLC) immediately for separation and quantitation of metabolites. Other methods for determining the level of metabolism are also acceptable and included herein.

The present invention also relates to monoclonal antibodies specific for cytochrome P450 2C9 (MAbs2C9).

An embodiment of the present invention is a monoclonal antibody specific for cytochrome P450 2C9 which was developed by the fusion of myeloma tumor cells with isolated B-lymphocytes from spleens of mice immunized with baculovirus-expressed human CYP2C9.

Another embodiment of the present invention is a monoclonal antibody specific for human CYP2C9 of the isotype IgM.

A preferred embodiment of the present invention is a monoclonal antibody specific for human CYP2C9 of the isotype IgM wherein the antibody exhibits cross-reactivity with CYP2C8 and, to a lesser extent, CYP2C19.

A further embodiment of the instant invention is a monoclonal antibody specific for human CYP2C9 of the isotype IgM which exhibits cross-reactivity with human CYP2C8 and CYP2C19 but does not cross-react with human CYP1A2, CYP2A6, CYP2B6, CYP2D6, CYP2E1, CYP3A4, CYP3A5 or CYP3A7. Two antibodies in accordance with this description - MAb 30-12-1 and MAb 73-19-2 - were deposited with the ATCC in accordance with the Budapest Treaty on August 20, 1999 and assigned accession numbers PTA-568 and PTA-569, respectively.

Especially preferred embodiments are monoclonal antibodies specific for human CYP2C9 as described above wherein the antibody inhibits at least about 70%, preferably, greater than 90% of human CYP2C9 activity, or more preferably, about 95% of human CYP2C9 activity (e.g., flurbiprofen 4'-hydroxylation).

Particularly preferred embodiments are monoclonal antibodies specific for human CYP2C9 as described above wherein the antibody inhibits at least about 90% of human CYP2C8 activity, or preferably, about 95% of CYP2C8 activity (e.g., taxol 6α-hydroxylation).

Further embodiments are monoclonal antibodies specific for human CYP2C9 as described above wherein the antibody inhibits at least about 20%, preferably 24% (MAb73-19-2), more preferably, at least about 40%, most preferably 46% (Mab 30-12-1), of CYP2C19 activity (mephenytoin 4'-hydroxylation).

Another embodiment of the present invention is a hybridoma developed by the fusion of myeloma tumor cells with isolated B-lymphocytes from spleens of mice immunized with insect cell microsomes comprising baculovirus-expressed antigen. Immunization involves injecting the antigen (in this case, the microsomal preparation) into an antibody-producing species, preferably a mammal and, more preferably, a mouse. Usually an initial injection is followed by subsequent booster injections to maximize the response. The antigen can be any substance which is capable, under appropriate conditions, of inducing a specific immune response. The amount of antigen injected must be adequate to elicit a sufficient amount of antibody to be detectable. In particularly preferred embodiments, Balb/c mice are immunized intraperitoneally with 50 µg of Sf21 cell microsomes containing baculovirus-expressed antigen (of approximately 29% purity) emulsified in 0.2 mL of complete Freund's adjuvant (first immunization), followed by two booster injections with incomplete Freund's adjuvant on the 10^{th} and 20 ^{th} days. Following immunization, the animal will produce B-cells that produce and secrete antibodies specific for the antigen. These cells can be isolated by removal of the spleen of said animal. Fusion of the antibody-producing cells to a suitable stable and long living cell line (i.e., immortal cell line such as myeloma tumor cells) is done according to techniques that are known in the art. Most preferably, fusion is performed with PEG 5000 as described in (Park et al., 1986 Biochem. Pharmacol. 35:2859-2867).

Particularly preferred is a hybridoma in accordance with the above description wherein the insect used is *Spodoptera frugiperda*.

A preferred embodiment of the instant invention is a hybridoma which produces monoclonal antibodies in accordance with the instant invention and which is made by the fusion of myeloma tumor cells with isolated B-lymphoctyes from spleens of mice immunized with insect cell microsomes comprising baculovirus-expressed human CYP3A4 (or human CYP2C9). Screening techniques to select a hybridoma colony consisting of cells that produce the desired antibody are known in the art. Preferably, positive clones are selected by comparison with microsomes of cells infected with wild type baculoviruses, the positive clones evidenced by a notable increase in absorption at 405 nm. In particularly preferred embodiments, selected positive hybridomas are then transferred to 24-well plates for further growth until confluence and the media tested for ELISA and inhibitory activity by HPLC or LC-MS for assurance of the desired MAbs excreted from cloned hybridomas. Hybridoma cells producing such inhibitory MAbs are then subcloned at least three times and the media tested at each time.

Another preferred embodiment involves a method of making hybridomas producing monoclonal antibodies specific for a particular antigen which comprises immunizing mice with microsomal proteins comprising baculovirus-expressed antigen, and fusing isolated B-lymphocytes from spleens of the immunized mice with myeloma tumor cells.

Yet another preferred embodiment is a method of making ascites fluid comprising monoclonal antibodies specific for a particular antigen which comprises immunizing mice with microsomal proteins comprising baculovirus-expressed antigen, fusing isolated B-lymphocytes from spleens of the immunized mice with myeloma tumor cells, identifying those hybridomas producing antibodies which are reactive to the antigen and which inhibit reactions catalyzed by the antigen, injecting the hybridomas into mice, and extracting the ascites fluid. Means by which one of ordinary skill in the art can identify antibodies reactive to the antigen are available in the art and well known, e.g., ELISA, Western blot, etc. Methods of determining whether an antibody is capable of inhibiting a particular reaction catalyzed by its respective antigen are also known in the art and involve carrying out the reaction both in the presence and in the absence of the antibody and determining the levels of substrate and product produced, the level of product produced indicating the level of metabolism had (i.e., a lower level of substrate-to-product conversion in the sample comprising the antibody indicating the presence of inhibition).

A further embodiment is a method of determining expression levels of cytochrome P450 3A4 (, cytochrome P450 2C9, or cytochrome P450 2C8) in a sample which comprises contacting the sample purportedly containing cytrochrome P450 3A4 (,CYP2C9 or CYP2C8, respectively) with the above antibody specific to the cytochrome used having a detectable label, and measuring the amount of label in said sample; said amount indicating the level of expression of CYP3A4 (,CYP2C9 or CYP2C8) in the sample. Detectable labels are labels which can be detected. Labels suitable in this process are known in the art and include fluorescent labels, radioactive labels, and enzymes wherein a substrate is metabolized to a quantifiable product (e.g., wherein the enzyme is alkaline phosphatase and the substrate is 5-bromo-4-chloro-3-indolyl-phosphate/nitroblue tetrazolium).

A preferred method of determining expression levels of cytochrome P450 3A4 (, cytochrome P450 2C9 or cytochrome P450 2C8) in a sample comprises separating a sample on a gel such as an SDS polyacrylamide gel, transferring same to a piece of material capable of adsorbing the separated sample (e.g., nitrocellulose paper), contacting the separated sample on the material with a first antibody in accordance with the above description specific for said cytochrome, contacting the material with a second antibody specific for the first antibody which comprises a detectable label (see above) allowing for the quantitation of the bound first antibody (e.g., an alkaline phosphatase-conjugated anti-mouse IgG (H+L) for CYP3A4, or an alkaline phosphatase-conjugated anti-mouse IgM (µ chain) for CYP2C8/9); and quantitating the level of antibody, same indicating the level of antigen. This last step would include, for example, treating the alkaline phosphatase-conjugated anti-mouse IgG (H+L) (or IgM (µ chain) for CYP2C8/9) on the adsorbed material with a substrate for the alkaline phosphatase, e.g., 5-bromo-4 chloro-3-indolyl-phosphate/nitroblue tetrazolium, the metabolism of same being identified by a colored product which is to be quantitated to determine the level of antigen.

Another embodiment is a method of identifying CYP3A4-(, CYP2C8- or CYP2C9-)catalyzed metabolism of a substrate, which comprises contacting a sample comprising both CYP3A4 (, CYP2C8 or CYP2C9) and the above antibody with the substrate, measuring both substrate and product levels, and comparing the substrate and product levels with levels obtained from a control sample comprising both CYP3A4 (, CYP2C8 or CYP2C9) and the substrate but not the antibody; a lower level of substrate-to-product conversion in the sample comprising the antibody indicating the presence of CYP3A4-(CYP2C8- or CYP2C9-)catalyzed metabolism. Thus, inhibition of CYP3A4-(, CYP2C8- or CYP2C9-)catalyzed metabolism by the antibody is present when the level of substrate-to-product conversion is less in the sample comprising the antibody; "substrate-to-product conversion" being the metabolism of the substrate to the product.

Yet another embodiment is a method of quantifying CYP3A4-specific (, CYP2C8-specific or CYP2C9-specific) metabolism of a substrate in a sample which comprises contacting a sample comprising both the above antibody specific to the cytochrome employed and cytochrome P450s known to be involved in the metabolism of the substrate with the substrate, measuring substrate and product levels, and comparing the substrate and product levels with levels obtained from a control sample comprising CYP3A4 (, CYP2C8 or CYP2C9) and the substrate but not the antibody. The degree of variation, in this case, indicates the contribution of CYP3A4 (, CYP2C8 or CYP2C9) to the metabolism of that particular substrate in the tissue preparation studied, be it human or other. "CYP3A4-specific metabolism" (, "CYP2C8-specific metabolism" or "CYP2C9-specific metabolism") in this respect, thus, means only that metabolism wherein CYP3A4 (, CYP2C8 or CYP2C9, respectively) is the responsible agent, and not other CYP3A4s which may be present in the sample. To enhance this method, antibodies to other CYP3A4s could also be used either in conjunction with the above antibodies or separate. In this manner, the particular contributions of each cytochrome P450 could be quantified and analyzed. This is extremely useful in situations where a compound is metabolized by more than one P450.

### BRIEF DESCRIPTION OF THE DRAWINGS

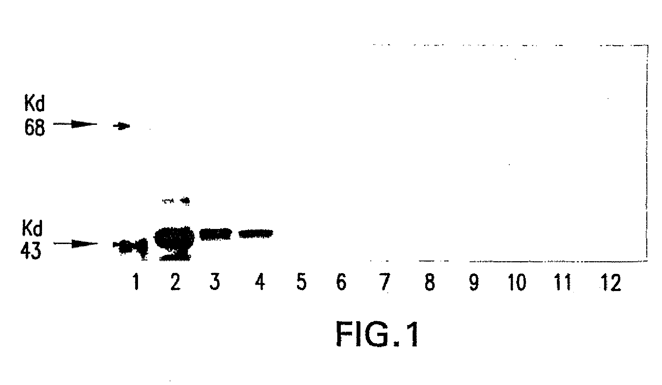

Figure 1 shows cross-reactivity of MAb_{3A4a} towards eleven recombinant P450s by western immunoblotting. Each lane contained 1 pmol of P450. Lane 1: molecular markers; lane 2: CYP3A4; lane 3: CYP3A5; lane 4: CYP3A7; lane 5: CYP1A2; lane 6: CYP2A6; lane 7: CYP2B6; lane 8: CYP2C8; lane 9: CYP2C9; lane 10: CYP2C19; lane 11: CYP2D6 and lane 12: CYP2E1.

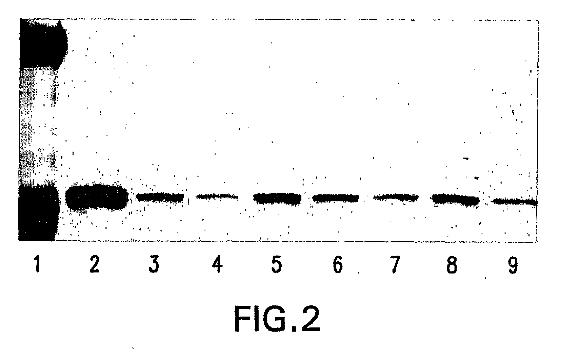

Figure 2 shows westernblot analyses of CYP3A4/5 in liver microsomes from seven individuals. 1 pmol of total P450 from each sample was loaded on the gel. Lane 1: molecular markers; lane 2: baculovirus-expressed CYP3A4; lane 3: L14; lane 4: L15; lane 5: L16; lane 6: L17; lane 7: L21; lane 8: L22 and lane 9: PH (pooled liver microsomes).
Figure 3 shows westernblot analyses of P450 present in liver microsomes from human, Rhesus monkeys, dogs, rats and mice. 1 Pmol of CYP3A4 and 5 pmol of microsomal P450s in human and animal livers were loaded on the gel. Lanes 1 and 9: molecular markers; lanes 2 and 8: CYP3A4; lane 3: human; lane 4: monkey; lane 5: dog; lane 6: rat and lane 7: mouse.
Figure 4 shows mAb_{3A4a} inhibition of testosterone metabolism by baculovirus-expressed CYP3A4, CYP3A5 and CYP3A7.
Figure 5 shows mAb_{3A4a} inhibition of diazepam metabolism catalyzed by recombinant CYP3A4.
Figure 6 shows inhibition comparison of MAb_{3A4a} with MAb-A334™ in the metabolism of testosterone catalyzed by recombinant CYP3A4.
Figure 7 shows the specificity of MAb_{3A4a} inhibition with twelve recombinant P450s.
Figure 8 shows the cross-reactivity of MAb_{3A4a} inhibition with P450-specific reactions catalyzed by human liver microsomes.
Figure 9 illustrates the metabolic pathways of diazepam by cytochrome P450s.

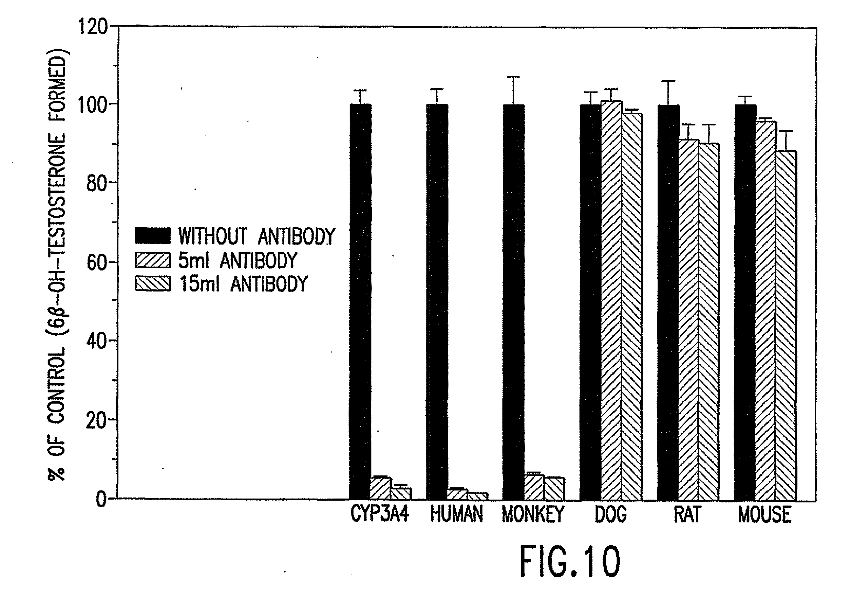

Figure 10 shows the effect of MAb_{3A4a} (0, 5 and 15 µl, blackened, grayed and white blocks, respectively) on testosterone-6β-hydroxylation catalyzed by recombinant CYP3A4, human, monkey, dog, rat and mouse liver microsomes.
Figure 11 shows an assessment of CYP3A and CYP2C contribution to diazepam 3-hydroxylation in liver microsomes of five samples.
Figure 12 shows an assessment of CYP3A and CYP2C contribution to diazepam *N*-demethylation in liver microsomes of five samples.
Figure 13 shows the analyses of MAb30-12-1 cross-reactivity towards nine recombinant P450s by Western immunoblotting. Each lane contained 1 pmol of P450. Lane 1: molecular markers; lane 2: CYP1A2; lane 3: CYP2A6; lane 4: CYP2B6; lane 5: CYP2C8; lane 6: CYP2C9; lane 7: CYP2C19; lane 8: CYP2D6; lane 9: CYP2E1; lane 10: CYP3A4; lane 11: wildtype microsomes; and lane 12: molecular markers.
Figure 14 shows the analyses of MAb73-19-2 cross-reactivity towards nine recombinant P450s by Western immunoblotting. Each lane contained 1 pmol of P450. Lane 1: molecular markers; lane 2: CYP1A2; lane 3: CYP2A6; lane 4: CYP2B6; lane 5: CYP2C8; lane 6: CYP2C9; lane 7: CYP2C19; lane 8: CYP2D6; lane 9: CYP2E1; lane 10: CYP3A4; lane 11: wildtype microsomes; and lane 12: molecular markers.
Figure 15 shows MAb30-12-1-mediated inhibition of flurbiprofen 4'-hydroxylation, taxol 6α-hydroxylation, and (-)mephenytoin 4'-hydroxylation by baculovirus-expressed CYP2C9, CYP2C8 and CYP2C19, respectively.
Figure 16 shows MAb73-19-2-mediated inhibition of flurbiprofen 4'-hydroxylation, taxol 6α-hydroxylation, and (-)mephenytoin 4'-hydroxylation by baculovirus-expressed CYP2C9, CYP2C8 and CYP2C19, respectively.
Figure 17 shows the specificity of MAb30-12-1-mediated inhibition of the metabolism of marker substrates catalyzed by individual recombinant P450s. The error bars represent a standard error.
Figure 18 shows the specificity of MAb73-19-2-mediated inhibition of the metabolism of marker substrates catalyzed by individual recombinant P450s.
Figure 19 shows an assessment of the contribution of CYP2C9 present in human liver microsomes to flurbiprofen 4'-hydroxylation probed with MAb30-12-1 and MAb73-19-2.
Figure 20 shows an assessment of the contribution of CYP2C9 and flurbiprofen 4'-hydroxylase contained in the liver microsomes from human, monkey, dog, rat and mouse to flurbiprofen 4'-hydroxylation with MAb30-12-1 and MAb73-19-2.

### DETAILED DESCRIPTION OF THE INVENTION

As used throughout the specification and claims, the following definitions shall apply:
The terms "specific" or "specificity" as used herein regarding antibodies against human CYP3A4 mean that these antibodies do not cross-react with the major human cytochrome P450 isoforms in the 1A, 2A, 2C, 2D, 2E, 3A subfamilies.
The terms "substantially inhibit", "inhibit", etc. as used herein regarding antibodies against human CYP3A4 mean that these antibodies inhibit at least about 80-95% of human CYP3A4 activity *in vitro.*
The terms "MAb_{3A4a}" and "MAb10-1-1" and "PTA-351" are synonymous for Applicants' monoclonal antibody as described herein.

A monoclonal antibody specific for cytochrome P450 3A4 (MAb_{3A4a}) of the isotype IgG₂ₐ has been identified. MAb_{3A4a} is directed at a single epitope on the antigen, cytochrome P450 3A4, allowing for the high specificity noted of monoclonal antibodies.

In order to obtain the above antibody, microsomes of *Spodoptera frugiperda* (Sf21) insect cells infected with recombinant baculoviruses encoding human CYP3A4 cDNA were used to immunize mice. Spleen cells from the immunized mice were then fused to myeloma cells, and hybridoma cells producing the monoclonal antibodies of the instant invention were identified using ELISA; identification being based on binding to microsomes containing CYP3A4 antigen. This involved subtracting out those non-specific antibodies with absorption at 405nm. Positive hybridoma clones were then tested against testosterone metabolism (6β-hydroxylation) to identify those clones effective in inhibiting CYP3A4-catalyzed testosterone metabolism. Following subcloning to obtain monoclonality, the resulting hybridoma cells of interest were injected into mice for the preparation of ascites fluid. Resultant ascites fluid exhibited a strong binding activity on immunoblot with CYP3A4 , with a lesser response to CYP3A5 and CYP3A7. MAb_{3A4a} did not cross-react with any of the other eight human P450s tested (CYP1A2, CYP2A6, CYP2B6, CYP2C8, CYP2C9, CYP2C19, CYP2D6, and CYP2E1).

CYP3A4 within insect microsome preparations served as both immunogen and cloning component of the hybridoma technology. The implications arising from this finding are significant. Applicants have found that, through the use of relatively impure antigen preparations in both immunization and cloning, one could obtain MAbs to P450 proteins which may be present at low concentrations or minor deviants of the primary protein in the immunogen preparation. Further, this method is not limited to antibodies of P450 molecules. Antibodies to other molecules of interest could be identified via this process as well. Accordingly, the previously reported procedures involving purification of large amount of P450 proteins in order to isolate MAbs are no longer necessary (Gelboin *et al*., 1995; Gelboin et al., 1997 Pharmacogenetics 7:469-477).

The resulting antibody in the instant case, MAb_{3A4a}, was shown to have both a strong ELISA and western blot with CYP 3A4 antigen. The positive westernblot result indicates that the epitope on CYP3A4 recognized by MAb_{3A4a} is of the native conformation of the protein. MAb_{3A4a} can thus be used beneficially as a sensitive probe for the quantitation of the specific CYP3A4 in human tissue with a sensitivity of 0.1 pmol of CYP3A4 being able to be reached on westernblot using alkaline phosphatase-conjugated anti-mouse IgG (H+L).

The antibody of the instant invention possesses many advantages over that disclosed in the art. For instance, roughly 80 µL of MAb-A334™ mouse ascites (Gelboin) per mL incubation is required to obtain inhibition of CYP3A4-mediated testosterone-6β-hydroxylation by 79%. By contrast, only 0.5 µL of MAb_{3A4a} ascites, a preparation of Applicants' monoclonal antibody, is required to achieve that percent inhibition (82%).

The antibody of the instant invention cross-reacts to a lesser extent with CYP3A5 and CYP3A7. This most likely reflects truly shared antigenic determinants on the surface of the antigens, especially given the highly conserved homology amongst CYP3A5/7 and CYP3A4, 84% and 88%, respectively. A monoclonal antibody like that of the instant invention which cross-reacts with CYP3A7 is a benefit for identification of CYP3A7 in human fetal liver in which CYP3A7 is a sole CYP3A subfamily member. Further, cross-reactivity of MA_{b3A4a} with CYP3A5 can be utilized to assess quantitatively the total contribution of CYP3A subclass to *in vitro* drug metabolism, although the expression level of CYP3A5 in liver is insignificant.

Notably, MAb_{3A4a} immunoblots microsomal P450(s) and strongly inhibits testosterone 6β-hydroxylase activity present in humans and monkey, but not in dog, rat, or mouse. The cross-reactivity of MAb_{3A4a} in closely related species is probably attributed to the highly conserved CYP3A sequences between human and monkey, in which CYP3A4 exhibits 93% identity of amino acid sequence with CYP3A8 (monkey), but to a lesser extent, 80% with CYP3A12 (dog), 73% and 72% with CYP3A1/2 in rat, and 73% with CYP*3a11* in mouse. Thus, MAb_{3A4a} directed against one CYP3A4 generally cross-reacts not only with other forms (CYP3A5 and CYP3A7) in the same species but also with other CYP3A forms in monkey. Consequently, cross-reactivity of the MAb with other species can provide an additional usefulness in the identification of CYP3A(s).

MAb_{3A4a} is, further, uniquely suited for reaction phenotyping, i.e., determining the contribution of epitope-specific CYP3A to metabolic reactions and for immuno-quantitation of CYP3A expression in human tissues. In addition, the cross-reactivity of MA_{b3A4a} with other CYP3As in both inhibitory and westernblotting activity can be very useful for the study of CYP3A7 in human fetal and CYP3A in monkey.

Monoclonal antibodies specific for cytochrome P450 2C9 (MAbs2C9) were also identified in accordance with the instant invention. Preferably, these monoclonal antibodies are of the isotype IgM. More preferably, the antibodies are Mabs 30-12-1 and 73-19-2, ATCC accession numbers PTA-568 and PTA-569, respectively. The antibodies were obtained in accordance with the methods described above except that instead of CYP3A4 cDNA, baculovirus-encoded CYP2C9 cDNA was employed. ELISA was carried out with microsomes containing CYP2C9 antigen. Also, selected hybridoma clones were tested for inhibition of CYP2C9-catalyzed flurbiprofen metabolism.

MAb 73-19-2 had a strong immunoblot with both CYP2C8 and CYP2C9. The binding of MAb 30-12-1 was found to be much more selective to CYP2C8 than CYP2C9. Both MAbs can be used to measure the expression levels of CYP2C8 and CYP2C9 present in a human tissue preparation. In accordance with the method of determining expression levels of CYP2C8/9 described above, the two MAbs (73-19-2 and 30-12-1) can be used simultaneously. This process would involve separating a sample separately on two gels; transferring the separated samples to a piece of material capable of adsorbing said samples; contacting the sample on the first gel with MAb 73-19-2; contacting the sample on the second gel with MAb 30-12-1; contacting both gels with a second antibody specific for the first antibody which comprises a detectable label allowing for the quantitation of the bound first antibody on each sample; and comparing the pattern of antibody detected on each gel. Given that MAb 73-19-2 has a strong immunoblot with both CYP2C8 and CYP2C9 and MAb 30-12-1 is much more selective to CYP2C8, only exhibiting a weak signal with CYP2C9, the two samples can be compared and the individual expression levels of CYP2C8 and CYP2C9 determined by comparison.

In terms of inhibition of CYP2C8/9-catalyzed reactions, ten µl of the MAb mouse ascites comprising the MAbs 30-12-1 and 73-19-2 in one mL incubation containing 25 pmol of CYP2C9 inhibited strongly the flurbiprofen 4'-hydroxylation by 96% and 97%, respectively and, to a same extent, cross-inhibited CYP2C8 activity, a member of CYP2C subfamily in taxol 6α-hydroxylation. The two Mabs cross-inhibited partially CYP2C19-mephenytoin 4'-hydroxylation by 46% and 24%, respectively. However, the Mabs exhibited no cross-reactivity with any of the other recombinant human P450 isoforms (CYP1A2, CYP2A6, CYP2B6, CYP2D6, CYP2E1, CYP3A4, CYP3A5 and CYP3A7) in the course of both cytochrome P450 reaction phenotyping and Western immunoblot analyses.

The Mabs were employed to assess the quantitative role of CYP2C9 to the flurbiprofen 4'-hydroxylation in liver microsomes. The results showed that CYP2C9 contributes 73-97% to flurbiprofen 4'-hydroxylation in human livers. In addition, flurbiprofen 4'-hydroxylase activity was inhibited almost completely in Rhesus monkey liver microsomes and inhibited partially in mouse by the two Mabs. By comparison, no significant inhibition of the enzyme was observed in the presence of rat and dog liver microsomes. The results demonstrate that inhibitory and immunoblot MAbs can offer a precise and useful tool for quantitative identification of CYP2C8/9 in the metabolism of clinically used drugs and drugs in development. In addition, the cross-reactivity of the MAb inhibition with the P450s in other species, i.e., monkey, can be used to assess the P450 contribution to the metabolism of a given drug or drug candidate in animal.

The following non-limiting examples are presented to better illustrate the invention.

### EXAMPLE 1

### Chemicals

The following chemicals were obtained from commercial sources: (±)-4-hydroxymephenytoin, (±)-mephenytoin, 6-hydroxychlorzoxazone, (±)-bufuralol hydrochloride salt, and (±)-1'-hydroxybufuralol maleate salt from ULTRAFINE Chemicals (Manchester, UK); diazepam (DZ), temazepam (TMZ), nordiazepam (NDZ), flurbiprofen, chlorzoxazone, taxol, coumarin, 7-hydroxycumarin, phenacetin, acetaminophen, and NADPH from Sigma (St. Louis, MO); testosterone, 6β-hydroxytestosterone, estrone, 2-hydroxyestrone, 6β-hydroxyprogesterone from Steraloids Inc. (Wilton, NH); MAb-A334™ (Cat. No =A334, anti-CYP3A4MAb), human CYP3A5+OR supersomes, 6-hydroxychlorzoxazone and 6α-hydroxypaclitaxol from Gentest Corp. (Woburn, MA); benzo[α]pyrene *trans*-4,5-dihydrodiol (B[*a*]P t-4,5-diol), phenanthrene, 9-hydroxyphenanthrene and benz[*a*]anthracene *trans*-5,6-dihydrodiol (BA *t*-5,6-diol) from the National Cancer Institute Chemical Carcinogen Repository (Kansas, MO); Sf-900II SFM, fetal bovine serum and HAT supplement from GIBCO (Frederick, MD); and 8-asaguanine resistant myeloma cell line, NS-1 from Frederick Cancer Research and Development Center (Frederick, MD).

### EXAMPLE 2

### Preparation of human P450s

Plasmids containing the full length cDNAs for P450s 3A4, 1A1, 1A2, 2A6, 2B6, 2C8, 2C9, 2C19, 2D6, 2E1, 3A5 and 3A7 and P450 oxidoreductase (OR) were provided from Dr. Frank J. Gonzalez of National Cancer Institute. These cDNAs are known in the art and are published as follows: Human CYP1A1 cDNA - Jaiswal et al., 1985 Science 228(4695):80-3, and Jaiswal et al., 1985 Nucleic Acids Res 13(12):4503-20; Human CYP1A2 cDNA - Jaiswal et al, 1986 Nucleic Acids Res 14(16):6773-4, and Jaiswal et al., 1987 J Exp Pathol 3(1):1-17; Human CYP2A6 cDNA- Yamano et al., 1990 Biochemistry 29(5):1322-9, and Yamano et al., 1989 Nucleic Acids Res 17(12):4888; Human CYP2B6 cDNA- Yamano et al., 1989 Biochemistry 28(18):7340-8; Human CYP2C8 cDNA - Kimura et al., 1987 Nucleic Acids Res 15(23):10053-4; Human CYP2C9 cDNA - Kimura et al., 1987 Nucleic Acids Res 15(23):10053-4; Human 2C19 cDNA - Romkes et al., 1991 Biochemistry 30, 3247-3277; Human CYP2D6 cDNA - Gonzalez et al., 1988 Nature 331(6155):442-6; Human CYP2E1 cDNA - Song et al., 1986 J. Biol. Chem. 261(35):16689-97 [published erratum appears in J Biol Chem 1987 Jun 25;262(18):8940]; Human CYP3A4 cDNA - Gonzalez et al., 1988 DNA 7(2):79-86; Human CYP3A5 cDNA - Aoyama et al., 1989 J. Biol. Chem. 264, 10388-10395; and Human CYP3A7 cDNA - Kitada et al., 1987 J. Biol. Chem. 262, 13534-13537.

The entire coding region of each cDNA was excised from the vectors by digestion with respective enzymes and inserted into baculovirus shuttle vector, pBlueBac 4.5 (Invitrogen Corp., Carlsbad, CA), downstream of the polyhedrin promoter. Recombinant virus was constructed according to the manufacturer's procedure (Gonzalez et al., 1991 Methods Enzymol. 206:93-99) and isolated using Blue-Gal for color selection of recombinant virus. After two runs of plaque purification, the recombinant baculoviruses were propagated in *Spodoptera frugiperda* (*Sf21*) cells to generate high titer virus stocks for protein expression. *Sf21* insect cells (Invitrogen) were grown at 27°C in complete Sf900 SFM medium (BRL, Gaithersburg, MD) to a density of 1-2 x 106 cells/mL (400 mL in total) in 1-liter spinner flasks (Bellco Glass, Inc., Vineland, NJ) with enlarged blades at 90 rpm. Cells were infected at approximately 1.0 MOI (multiplicity of infection) of virus encoding individual P450s and 0.1 MOI of virus encoding OR. 1 µg hemin/mL medium in the form of a hemin-albumin complex was added. After 72 hours, cells were harvested by centrifugation and resuspended in 20% glycerol in 0.1 M KPi (potassium phosphate buffer) and stored at -70°C until microsomal preparation. The total P450 content was measured by the CO-difference spectrum. Microsomes were prepared as described below and the resulting protein concentration was determined with bicinchoninic acid according to the manufacturer's directions (Pierce Chemical Co., Rockford, IL). The activities of individual P450s co-expressed with OR were determined by the assays as indicated in Table 1 below. The assays of Table 1 are known in the art and published as follows: P450s 3A4, 3A5, and 3A7 by Hanioka et al., 1990 Protein Eng. 3:571-575; P450 1A1 by Shou et al., 1994 Cancer Lett.83:305-313; P450 1A2 by Shou et al., 1997 Carcinogenesis 18:207-214; P450 2A6 by Yun et al., 1991 Mol. Pharmacol.40:679-685; P450 2B6 by Yang et al., 1998 Biochem. Pharmacol. 55:889-896; P450 2C8 by Rahman et al., 1994 Cancer Res.54:5543-5556; P450 2C9 by Tracy et al., 1995 Biochem. Pharmacol.49:1269-1275; P450 2C19 by Goldstein et al., 1994 Biochemistry33:1743-1752; P450 2D6 by Crespi et al., 1995 Pharmacogenetics 5:234-243; and P450 2E1 by Peter et al., 1990 Chem. Res. Toxicol.3:566-573.

**Table 1. Assays for individual P450s**

| P450^{a} | Substrate | Conc. (µM) | MAb (µl/mL)^{b} | P450 Conc. (nM) | Product | Internal Standard^{c} |
|---|---|---|---|---|---|---|
| 3A4 | Testosterone | 200 | 10 | 15 | 6β-OH-testosterone | 6β-OH-progesterone |
| 3A5 | Testosterone | 200 | 10 | 15 | 6β-OH-testosterone | 6β-OH-progesterone |
| 3A7 | testosterone | 200 | 10 | 15 | 6β-OH-testosterone | 6β-OH-progesterone |
| 1A1 | phenanthrene | 300 | 10 | 25 | 9,10-dihydrodiol | B[*a*]P 9,10-diol |
| 1A2 | estrone | 100 | 10 | 50 | 2-OH-estrone | B[*a*]P cis-4,5-diol |
| 2A6 | coumarin | 100 | 10 | 20 | 7-OH-coumarin | 7-ethoxycoumarin |
| 2B6 | diazepam | 200 | 10 | 50 | Nordiazepam | 2-oxo-quanzepam |
| 2C8 | taxol | 25 | 10 | 25 | 6α-OH-taxol | Baccatin III |
| 2C9 | flurbiprofen | 20 | 10 | 15 | 4'-OH-flurbiprofen | B[*a*]A 5,6-diol |
| 2C19 | mephenytoin | 100 | 10 | 25 | 4'-OH-mephenytoin | Nirvanol |
| 2D6 | bufuralol | 25 | 10 | 10 | 1'-OH-bufuralol | Temazepam |
| 2E1 | chlorzoxazone | 200 | 10 | 50 | 6-OH-chlorzoxazone | B[*a*]A 5,6-diol |

| | | | | | | |
|---|---|---|---|---|---|---|
| ^{a.} Baculovirus-expressed individual P450s were co-expressed with OR in Sf21 insect cells as described in the text. ^{b.} MAb concentration was expressed as µL of mouse ascites per mL incubation mixture. ^{c.} Internal standard was used for metabolite quantitation. | | | | | | |

### EXAMPLE 3

### Microsomal preparation from human, animal livers and Sf21 cells expressing individual P450s

Microsomes of *Sf21* cells, human, Rhesus monkey, dog, rat and mouse livers were prepared by two different centrifugations (9,000g and 105,000g) as described previously (Wang et al., 1983 Biochemistry 22:5375-5383) and were reconstituted in a buffer (pH=7.4) containing 0.25 M sucrose, 1 mM EDTA, 0.5 mM DTT, 1.15% KCl and 0.1M KPi and stored at -80°C until used. Sf21 cell microsomes containing individual P450s with and without OR were used as a source of enzyme for metabolism study and immunogens for MAb production, respectively.

### EXAMPLE 4

### Procedure for MAb development

Three female Balb/c mice were immunized intraperitoneally with 50 µg of Sf21 cell microsomes containing baculovirus-expressed human CYP3A4 (29% purity) protein emulsified in 0.2 mL of complete Freund's adjuvant (first immunization), followed by two booster injections with incomplete Freund's adjuvant on 10^{th} and 20^{th} days. Three days after the third injection, splenocytes of the mouse were taken for fusion with non-secreting myeloma cells P3/NSI/1-Ag4-1 (NS-1). Fusion of the spleen cells with the NS-1 cells was performed with PEG 5000 as previously described (Park et al., 1986 Biochem. Pharmacol. 35:2859-2867) and the fused cells were plated in microtiter wells in 96-well plate at a density of 1 x 10⁴ cells per well and HAT medium was supplemented with 20% of FBS PRMI 1640 medium. Microtiter wells were examined daily for hybridoma growth. Three weeks later, when hybridoma cells approached confluence, media were tested with ELISA using baculovirus-expressed CYP3A4 as antigen (0.1 pmol/well). Positive clones (a total of 128 out of 1000 wells) were selected by comparison with microsomes of cells infected with wild type baculoviruses, which showed an approximate 3-fold increase in absorption at 405 nm with CYP3A4 (vs. wild type protein). Selected hybridomas were transferred to 24-well plates for further growth until confluence and then media were further tested for ELISA and inhibitory activity by HPLC or LC-MS for assurance of the desired MAbs excreted from cloned hybridomas. Of the 128 clones, one hybridoma clone (Mab_{3A4a}) resulted in potent inhibition of CYP3A4-catalyzed 6β-hydroxylation of testosterone(>93%); procedure below. Hybridoma cells producing such inhibitory MAbs were subcloned at least three times and their media were tested at each time.

### EXAMPLE 5

### ELISA (Enzyme-Linked Immunosorbent Assay)

ELISA was used for testing the binding activity of hybridoma supernatants collected from hybridoma screening (Gelboin *et al*., 1995). This procedure utilized alkaline phosphatase-conjugated goat F(ab')2 fragment to mouse IgG (H+L) from Cappel Research Products, and to mouse IgG (γ chain specific) or to mouse IgM (µ chain specific) from Jackson Immuno-Research Laboratories. Immunoassay plates were coated with Sf 21 microsomes containing baculovirus-expressed BYP3A4 at 1 pmol/well (100µL) or wild type baculovirus infected Sf21 cell microsomes as control in 1 X coating solution. As the hybrids began to grow, the spent medium from each well was screened for the presence of antibody.

### EXAMPLE 6

### Immunoblot Assay

To determine the specificity and binding activity of MAbs, eleven baculovirus-expressed P450s and liver microsomes from human donors, Rhesus monkeys, dogs, rats and mice were subjected to SDS-PAGE on an 8% polyacrylamide gel for 2 hr at 120 volt (1 pmol/lane). Wild type Sf21 cell microsomes were also used as a negative control. The proteins were transferred to nitrocellulose filters using prechilled buffer containing 25 mM Tris, 200 mM glycine, and 20% methanol (pH=8). The nitrocellulose membranes were incubated for 2 hr in BSA blocking buffer (3% w/v BSA in TBS containing 0.05% v/v Tween-20) to reduce nonspecific binding and then washed twice for 5 min with TBS containing 0.05% v/v Tween-20. The ascites fluid containing MAbs was diluted in antibody buffer at a final titer of 1:1000 or 1:2000 and incubated with membrane for 3 hr at room temperature. The membranes were washed and protein bands were detected using alkaline phosphatase-conjugated anti-mouse IgG (H+L).

### EXAMPLE 7

### Production of mouse ascites containing Mab_{3A4a}

Large scale production of MAbs was performed by growing cloned hybridomas in culture. The MAb-forming hybridomas (1 x 106 cells/mouse) were injected intraperitoneally into pristine-primed female BALB/c mice for production of concentrated MAbs. The ascites fluid was built up and withdrawn, two to three weeks after inoculation, and stored at -70°C (Park et al., 1986 Biochem. Pharmacol. 35:2859-2867). The protein concentration of this immunoglobulin preparation was 74 mg/mL mouse ascites.

### EXAMPLE 8

### Isotyping of mouse immunoglobulin (Ig)

Isotyping of MAbs was conducted by the Ouchterlony immunodiffusion using the mouse monoclonal antibody typing kits containing anti-mouse IgG₁, IgG₂ₐ, IgG_{2b}, IgG₃ and IgM from Binding Site, Inc. (Birmingham, AL). The murine immunoglobulin isotype of the Mab_{3A4a} was identified as IgG₂ₐ.

### EXAMPLE 9

### MAb inhibition of P450-catalyzed metabolism

To determine inhibitory activity of the MAb, 10 µl of ascites (or diluted ascites containing inhibitory MAbs) was added to 950 µl of 100 mM KPi (potassium phosphate buffer, pH = 7.5) containing 10-50 pmol of each recombinant P450, or 50-100 pmol of total P450 from liver microsomes of human, monkey, dog, rat and mouse. The mixture was vortexed gently and was preincubated at room temperature for 5 min. The reaction was initiated by the addition of substrate(s) and NADPH (1 mM) in a total volume of 1-mL and incubated at 37°C for 15-30 min depending on substrates used. Incubations were terminated by the addition of 8 vol. of dichloromethane (DCM) and corresponding internal standards; see Table 1. The remaining substrate and metabolites formed were extracted and centrifuged for 10 min (500g). Organic phase was evaporated to dryness under a stream of nitrogen. The residue was dissolved in a mixture of methanol or acetonitrile and water and analyzed immediately by reverse-phase HPLC (HP1100) or LC-MS (Perkin Emer API-150). P450-catalyzed reactions were assayed with slight modifications; see Table 1. The modifications where needed were primarily to change the HPLC conditions including column selection and elution of the gradient to resolve better separation of the metabolites for quantitation.

The assays of phenanthrene-9-hydroxylation (CYP1A1), estrone 2-hydroxylation (CYP1A2), diazepam N-demethylation (CYP2B6), and testosterone 6β-hydroxylation (CYP3A4/5/7) were performed virtually as disclosed; Shou et al., 1994 Cancer Lett. 83:305-313; Shou et al., 1997 Carcinogenesis 18:207-214; Yang et al., 1998 Biochem. Pharmacol. 55:889-896; and Hanioka et al., 1990 Protein Eng. 3:571-575, respectively. For all other assays, separation of metabolites formed was accomplished using a Hewlett Packard model HP1100 liquid chromatograph. The metabolite of taxol 6α-hydroxylation (CYP2C8) was isolated on an ODS Hypersil column (5µ, 4.6 mm x 20 cm, Hewlett Packard) and detected at a UV absorption of 230 nm; Rahman et al., 1994 Cancer Res. 54:5543-5556. The mobile phase for metabolite elution was 10% acetonitrile (ACN) in water for 5-min isocratic and then a 25-min gradient to 65% ACN in water. The metabolite of bufuralol 1'-hydroxylation (CYP2D6) was separated by Zorbax C8 column (5µ, 4.6mm x 15 cm), eluted with 20% ACN in water for 5 min and a 15-min gradient from 20% to 70%, and detected with a fluorescence detector at 252 nm of excitation and 302 nm of emission; Crespi et al., 1995 Pharmacogenetics 5:234-243. The 4'-hydroxy product formed in the metabolism of flurbiprofen was measured on the ODS C18 20/20 column (5µ, 4.6mm x 15 cm) and eluted with a 20-min gradient from 30% ACN in water containing 0.1% acetic acid to 60% at a detection of 260 nm of excitation and 320 nm of emission; Tracy et al., 1995 Biochem. Pharmacol. 49:1269-1275. 7-Hydroxycoumarin, a metabolite of coumarin 7-hydroxylation (CYP2A6) was analyzed by HPLC on a column (ODS C18 20/20 column, 5µ, 4.6mm x 15 cm) eluted with a gradient from 40% methanol in water to 70% under a detection of 320 nm; Yun *et al*., 1991 *Mol*. *Pharmacol*.40:679-685. Separation of mephenytoin 4'-hydroxylation (CYP2C19) was performed by using ODS-C18 20/20 column which eluted with a 20-min gradient from 10% ACN in water containing 0.1 % acetic acid to 40% and the metabolite was detected by UV absorption of 222 nm; Goldstein et al., 1994 Biochemistry 33:1743-1752. 6-Hydroxychlorzoxazone is a product of chlorzoxazone metabolism by CYP2E1 (Peter et al., 1990 Chem. Res. Toxicol. 3:566-573) and separated on ODS-C18 20/20 column using an elution of a 20-min gradient from solvent A (9% ACN:1 % tetrahydrofuran:90% water) to solvent B (79%CAN:1% tetrahydrofuran:9% water) and the detection was 287 nm. Flow rate of HPLC for all assays was 1 mL/min and the quantitation of metabolite was normalized the ratio of the metabolite to internal standard as listed in Table 1. Percentage inhibition of the MAb for the P450-catalyzed metabolism was determined by comparing the rate of metabolite formation between the absence and the presence of the MAb.

### EXAMPLE 10

### MAb_{3A4a} inhibition of CYP3A4/5/7-catalyzed metabolism

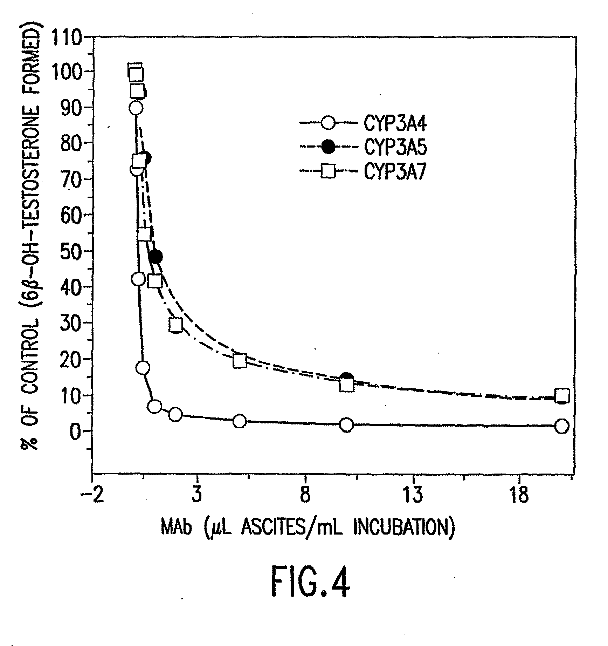

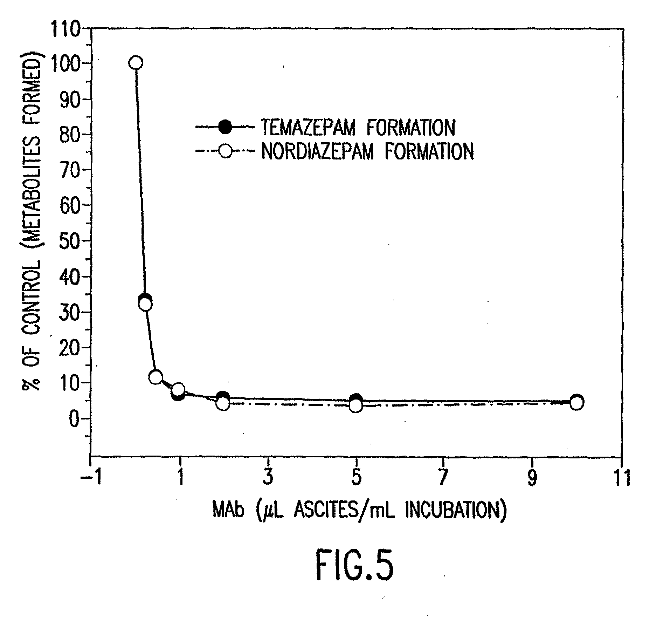

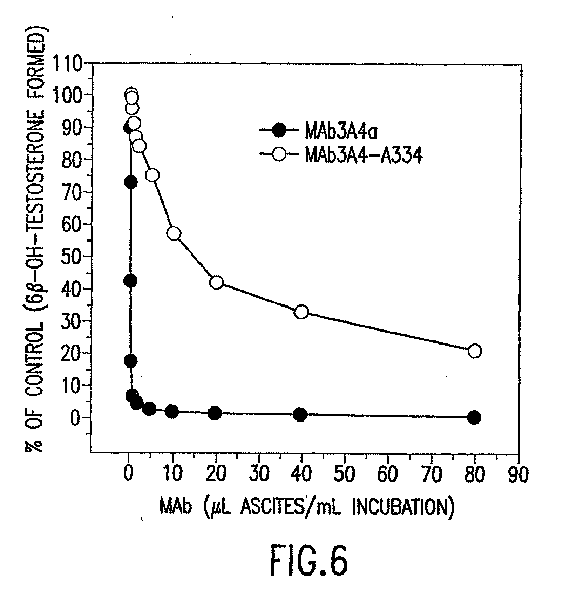

MAb_{3A4a} was examined for its inhibitory effect on CYP3A4-, CYP3A5-, and CYP3A7-catalyzed metabolism of two known substrates, testosterone and diazepam. Figs. 4 and 5 show the titration curve of MAb inhibition of metabolism versus mouse ascites amount. MAb_{3A4a} was found to be very inhibitory to all three CYP3A forms. As seen in Fig. 4, addition of MAb_{3A4a} at 1 µl ascites per one mL incubation of testosterone containing 20 pmol of each enzyme, inhibited CYP3A4/5/7-catalyzed testosterone 6β-hydroxylation by 95%, 52% and 58%, respectively, while at 10 µl/mL, metabolism was inhibited by 99%, 90% and 90%, respectively. Similarly, MAb_{3A4a} (10 µl) inhibited the CYP3A4-mediated conversion of diazepam (DZ) to temazepam (TMZ) and nordiazepam (NDZ) by 99% and 98%, respectively (Fig. 5). In contrast to MAb_{3A4a}, MAb-A334™ (Gentest Corp.) was employed to inhibit CYP3A4-mediated 6β-hydroxylation of testosterone. As seen in Fig. 6, MAb_{3A4a} and MAb-A334™ at 5 µl ascites in one mL incubation inhibited separately CYP3A4 activity by 99% and 24%. When 80 µL ascites of MAb-A334™ was used, only 79% inhibition was observed. These results demonstrate that MAb_{3A4a} is more potent than MAb-A334™.

### EXAMPLE 11

### Cross-inhibition with other human P450s

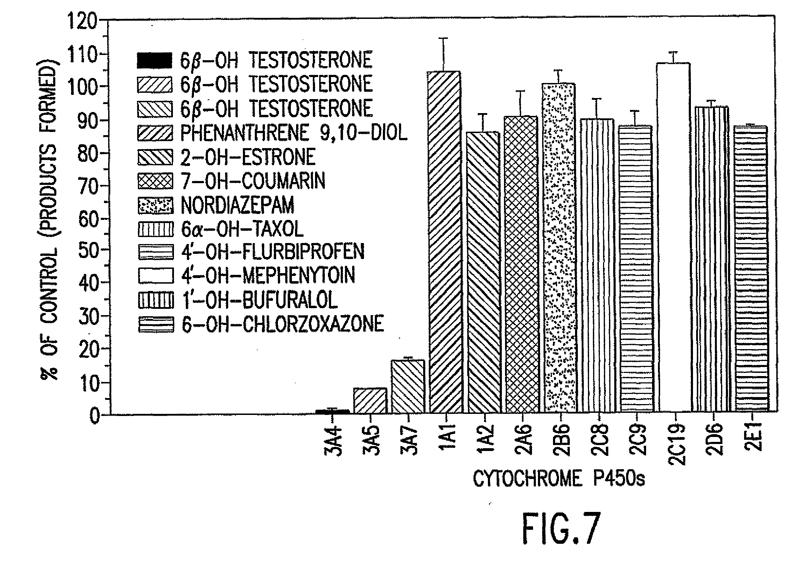

To confirm the specificity of the MAb_{3A4a}, assays for individual cDNA-expressed P450s were performed as shown in Table 1. 10 µl of MAb_{3A4a} ascites was added to one mL of incubation with individual P450s (15-30 pmol per incubation) to examine the cross-inhibition. Fig. 7 shows that MAb_{3A4a} inhibited strongly CYP3A4/5/7-catalyzed testosterone-6β-hydroxylation by 99%, 93% and 94%, respectively but did not display any significantly inhibitory cross-reactivity towards the cDNA-expressed human P450-mediated phenanthrene-9,10-epoxidation (CYP1A1), estrone-2-hydroxylation (CYP1A2), coumarin-7-hydroxylation (CYP2A6), diazepam-*N*-demethylation (CYP2B6), taxol-6α-hydroxylation (CYP2C8), flurbiprofen-4'-hydroxylation (CYP2C9), (S)mephenytoin-4'-hydroxylation (CYP2C19), bufuralol-1'-hydroxylation (CYP2D6) and chlorzoxazone-6-hydroxylation (CYP2E1), respectively, which represented a range of 86% to 108% of the enzyme activity in the absence of the MAb. The results suggest that these P450s may lack the epitope that is recognized by the MAb_{3A4a}.

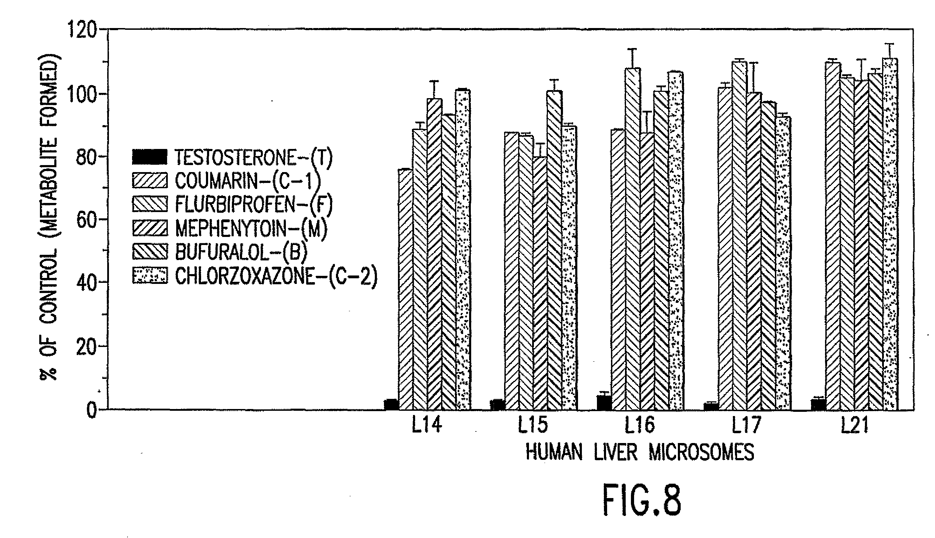

The selectivity of MAb_{3A4a} towards CYP3A4 was also evaluated by human liver microsomes (Fig. 8). Results showed that MAb_{3A4a} inhibited almost exclusively the testosterone-6β-hydroxylation (95∼98%), indicating that the 6β-position as a sole catalytic site for the CYP3A isoforms present in human liver. In contrast to testosterone assay, MAb_{3A4a} did not exhibit any significant effect on coumarin-7-hydroxylation (CYP2A6, 76∼110% of control), flurbiprofen-4'-hydroxylation (CYP2C9, 87∼110%), (S)mephenytoin-4'-hydroxylation (CYP2C19, 80∼104%), bufuralol-1'-hydroxylation (CYP2D6, 92∼115%) and chlorzoxazone-6-hydroxylation (CYP2E1, 89∼111%) (Fig. 8). The minor inhibition exhibited by the MAb_{3A4a} for some marker assays (up to 24%) could be contributed by the overlapping substrate specificity of CYP3A4 in human liver.

To determine whether MAb_{3A4a} inhibition was independent of substrate concentration [S], pooled human liver microsomes were used at varying substrate concentration (relative to Kₘ). Table 2 below shows that MAb_{3A4a} inhibited strongly the CYP3A4 activity (95∼98%) and had no effect on the reactions catalyzed by other P450s. These imply that the MAb-induced inhibition was insensitive to [S] and Kₘ at a saturating concentration of MAb_{3A4a.} As a result, MAb inhibition has an advantage over competitive chemical inhibitors, wherein the inhibitory specificity and potency often depend on the [S] and Kₘ of substrates studied.

**Table 2. MAb_{3A4a} inhibition of the metabolism of marker substrates by human liver microsomes**

| Substrate | Conc.^{a} (µM) | % Control^{b} (+ MAb) | Substrate | Conc. (µM) | % Control (+ MAb) |
|---|---|---|---|---|---|
| Testosterone (CYP3A4)^{c} | 250 | 1.97±0.05 | Bufuralol (CYP2D6) | 50 | 115.2±0.23 |
| | 75 | 2.60±0.00 | | 15 | 111.9±9.08 |
| | 25 | 4.55±0.06 | | 5 | 106.8±14.5 |
| Chlorzoxazone (CYP2E1) | 500 | 104.7±6.37 | Diclofenac (CYP2C9) | 100 | 108.3±1.16 |
| | 100 | 103.6±0.56 | | 30 | 113.1±1.14 |
| | 20 | 119.8±0.3 | | 10 | 125.7±0.15 |
| Mephenytoin (CYP2C19) | 400 | 99.5±1.41 | Phenacetin (CYP1A2) | 1000 | 101.9±1.75 |
| | 120 | 94.6±4.33 | | 150 | 107.5±1.12 |
| | 40 | 96.4±1.99 | | 25 | 113.9±0.91 |

| | | | | | |
|---|---|---|---|---|---|
| ^{a.} Concentration of substrate used for metabolism. ^{b.} % control was determined by a difference of activity between the presence and absence of MAb_{3A4a}. ^{c.} Individual P450 activities were probed with respective marker substrates. | | | | | |

### EXAMPLE 12

### Cross-inhibition in different species.

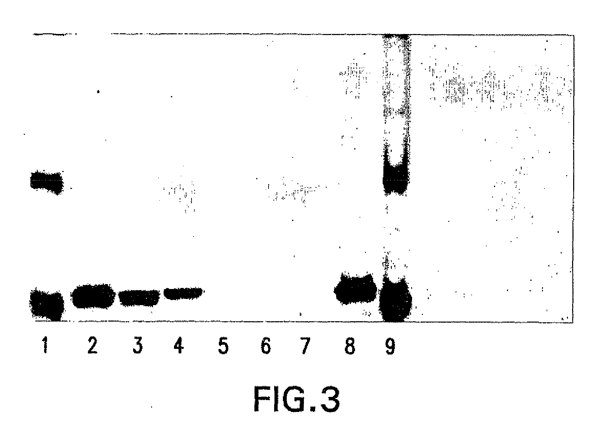

MAb_{3A4a} inhibited the testosterone 6β-hydroxylase activity (94-98%) in liver microsomes from human and Rhesus monkey. By comparison, no inhibition was observed with dog, rat and mouse liver microsomes. This was in agreement with the specificity of MAb_{3A4a} observed with immunoblot assay (Fig. 3).

### EXAMPLE 13

### Quantitative Assessment of CYP3A4/5 Contribution to the Metabolism of Substrates in Human Liver Microsomes.

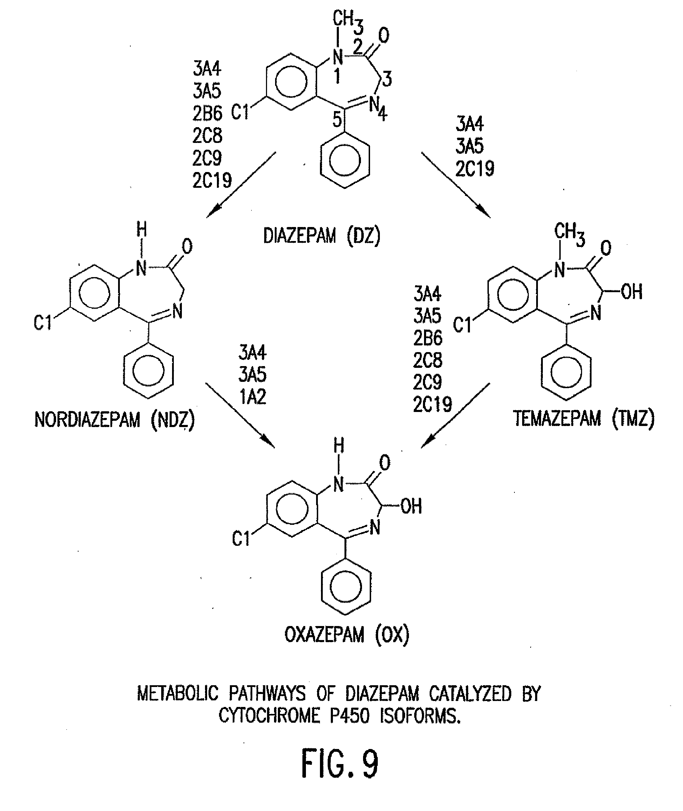

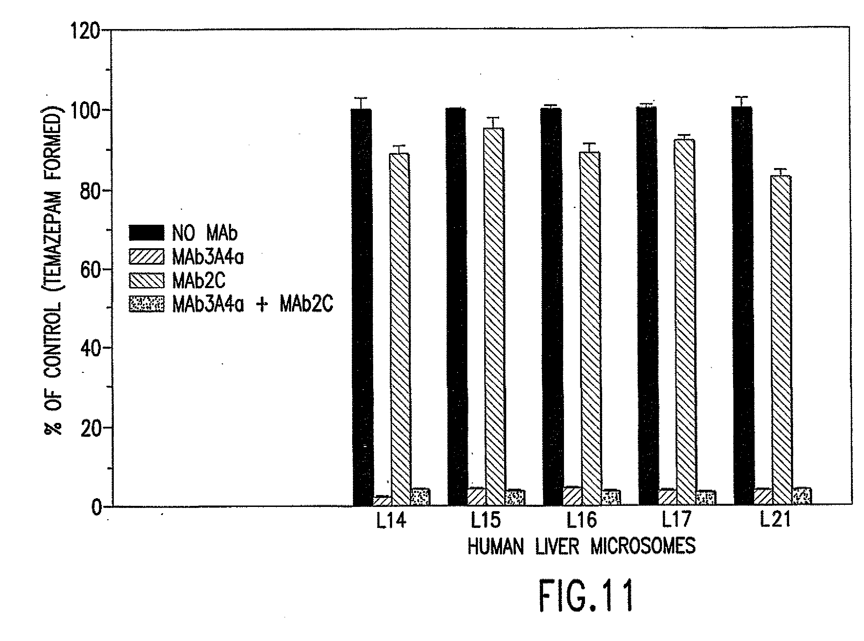

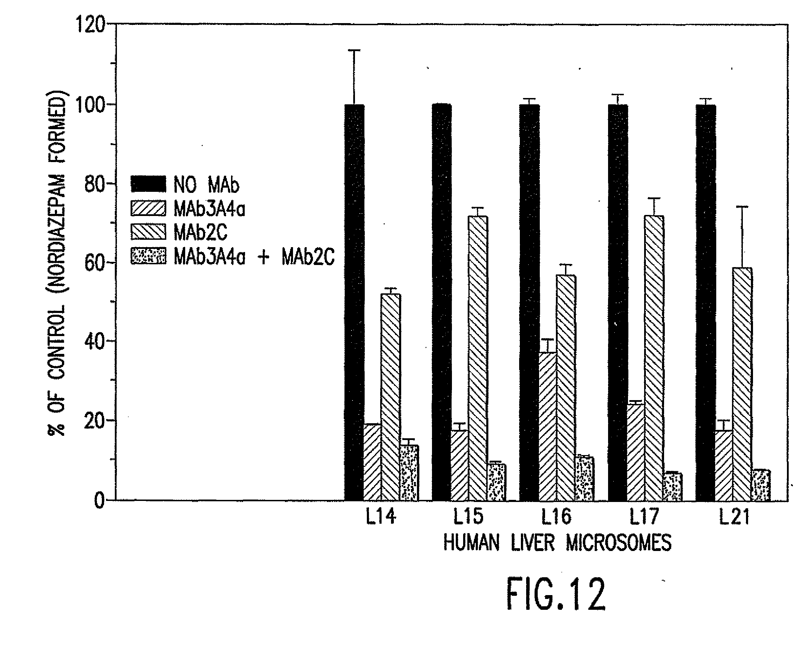

Testosterone is known to be a prototype substrate for CYP3A. MAb_{3A4a} inhibited testosterone 6β-hydroxy formation by more than 95% in all five microsomal preparations, indicating that CYP3A4/5 are essentially the sole P450 responsible for testosterone metabolism in human liver (Fig. 8). Diazepam (DZ) is hydroxylated to temazepam (TMZ) mainly by CYP3A4/5 and *N*-demethylated to nordiazepam (NDZ) by multiple P450s, (e.g., CYP3A4/5, 2B6 and 2C8/9/19) as illustrated in Figure 9. We utilized MAb_{3A4a} and MAb_{2C} (inhibitory anti-CYP2C8/9/19) MAb to assess the additive contribution of the CYP3A and CYP2C subfamilies to TMZ and NDZ formation in human livers. The data indicated that MAb_{3A4a} at 20 µl of ascites in 1-ml of liver microsomal incubation containing 100 pmol of P450 and 100 µM diazepam, inhibited the conversion of DZ to TMZ by >95% but MAb_{2C} had no apparent effect on TMZ formation (Fig. 11). This indicated that CYP3A4/5 are responsible for the 3-hydrozylation of diazepam in human liver (reaction-specific). However, when the production of nordiazepam was monitored, the reaction was inhibited partially by MAb_{3A4a} (52-73%) and MAb_{2C} (9-37%) (Fig. 12). Interestingly, an additive inhibition was observed in each of 5 liver microsomal samples when both Mabs were used together, showing that 86-93% of the *N-*demethylation of DZ relied on both CYP3A and CYP2C isoforms (Fig. 12). Thus, when a reaction is suspected to be catalyzed by multiple P450s, two or more Mabs can co-incubate in order to evaluate their combined contribution to the metabolism of a particular drug.

### EXAMPLE 14

### Procedure for MAb 2C9 development

Three female Balb/c mice were immunized intraperitoneally with 50-100 µg of Sf21 cell microsomes containing baculovirus-expressed CYP2C9 protein emulsified in 0.2 mL of complete Freund's adjuvant (first immunization), followed by two booster injections with incomplete Freund's adjuvant on the 10^{th} and 20^{th} days. Three days after the third injection, splenocytes of the mouse were taken for fusion with non-secreting myeloma cells P3/NSI/1-Ag4-1 (NS-1). Fusion of the spleen cells with the NS-1 cells was performed with PEG 5000 and the fused cells were plated in microtiter wells in 96-well plate at a density of 1 x 10⁴ cells per well. HAT medium was supplemented with 20% of FBS PRMI 1640 medium. Microtiter wells were examined daily for hybridoma growth. Three weeks later, when hybridoma cells approached confluence, media was tested with ELISA using baculovirus-expressed CYP2C9 as antigen (0.1 pmol/well). Positive clones (a total of 144 out of 1000 wells) were selected by comparison with microsomes of cells infected with wild type baculoviruses, which showed an approximate 2-fold increase or greater in absorption at 405 nm with CYP2C9 (vs. wild type protein). The background observed with the wild type microsomes can vary in a range of 0.1∼1.0 absorption unit per well due to the antibodies raised against non-specific microsomal proteins. Selected hybridomas were transferred to 24-well plates for further growth until confluence, and then the media was further tested for ELISA and inhibitory activity towards CYP2C99-catalyzed flurbiprofen metabolism by HPLC or LC-MS for assurance of the desired MAbs excreted from cloned hybridomas. Of the 144 clones, two hybridoma clones (MAb30-12-1 and MAb73-19-2) resulted in a potent inhibition of CYP2C9-catalyzed 4'-hydroxylation of flurbiprofen (>90%). These hybridoma cells producing inhibitory MAbs were subcloned at least three times and their media were tested at each time. The murine immunoglobulin isotype of the two Mabs was identified as IgM by the Ouchterlony immunodiffusion technique; see Example 8.

### EXAMPLE 15

### ELISA for MAbs 2C9

ELISA was used for testing the binding activity of hybridoma supernatants collected from hybridoma screening. Alkaline phosphatase-conjugated goat F(ab')2 fragment to mouse IgG (H+L) was purchased from Cappel Research Products, and to mouse IgG (γ chain specific) or to mouse IgM (µ chain specific) was from Jackson Immuno-Research Laboratories.

### EXAMPLE 16

### Immunoblot Assay for MAbs 2C9

To determine the specificity and binding activity of MAbs, nine baculovirus-expressed P450s (1A2, 2A6, 2B6, 2C8, 2C9, 2C19, 2D6, 2E1 and 3A4) were subjected to SDS-PAGE on an 8% polyacrylamide gel for 2 hr at 120 volt (1 pmol/lane). Wild type Sf21 cell microsomes were also used as a negative control. The proteins were transferred to nitrocellulose filters using prechilled buffer containing 25 mM Tris, 200 mM glycine, and 20% methanol (pH=8). The nitrocellulose membranes were incubated for 2 hrs in BSA blocking buffer (3% w/v BSA in TBS containing 0.05% v/v Tween-20) to reduce nonspecific binding and then washed twice for 5 min with TBS containing 0.05% v/v Tween-20. The ascites fluid containing MAbs 30-12-1 and 73-19-2 was diluted in antibody buffer at a final titer of 1:1000 or 1:2000 and incubated with membrane for 3 hrs at room temperature. The membranes were washed and protein bands were detected using alkaline phosphatase-conjugated anti-mouse IgM (H+L).

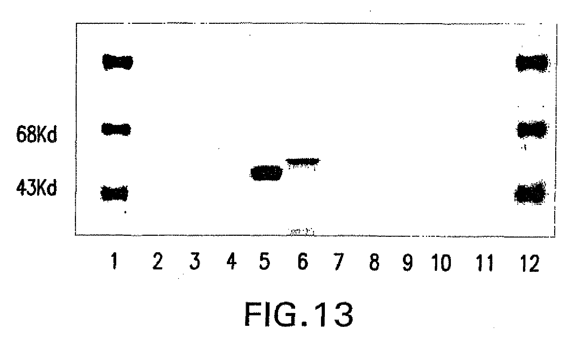

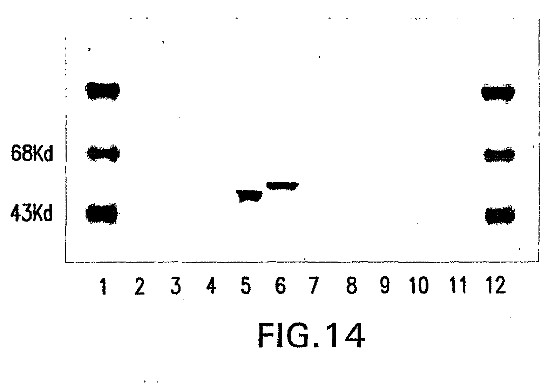

At a gel loading of 1 pmol, MAb30-12-1 exhibited a strong binding activity with CYP2C8 and to a much lesser extent, with CYP2C9; see Figure 13. MAb73-19-2, by contrast, showed binding activity to a same extent for both CYP2C8 and CYP2C9; Figure 14. The two Mabs are, therefore, not characteristically the same in immunoblotting activity. Interestingly, the Mabs did not cross-bind to CYP2C19, a member of the CYP2C subfamily. They, further, did not cross-react with any of the seven P450s from other families.

### EXAMPLE 17

### Production of Mouse Ascites containing Mabs 2C9

Large-scale production of MAbs was performed by growing cloned hybridomas in culture. The MAb-forming hybridomas (1 x 10⁶ cells/mouse) were injected intraperitoneally into pristine-primed female BALB/c mice for production of concentrated MAbs. The ascites fluid was built up and withdrawn, two to three weeks after inoculation, and stored at -70°C.

### EXAMPLE 18

### MAb Inhibition of P450-catalyzed Metabolism by Mabs 2C9

To determine the inhibitory activity of the MAb, 10 µL of ascites (or diluted ascites containing inhibitory MAbs) was added to 950 µL of 100 mM KPi (potassium phosphate buffer, pH = 7.5) containing 10-50 pmol of each recombinant P450, or 100 pmol of total P450 from liver microsomes of human, and 200 pmol from monkey, rat and mouse. The mixture was vortexed gently and was preincubated at room temperature for 5 min. The reaction was initiated by the addition of each substrate and NADPH (1 mM) in a total volume of 1-mL and incubated at 37°C for 15-30 min depending on substrates used. Incubations were terminated by the addition of 8 vol of dichloromethane (DCM) and respective internal standards (Table 1). The remaining substrate and metabolites formed were extracted and centrifuged for 10 min (500g). The organic phase was evaporated to dryness under a stream of nitrogen. The residue was dissolved in a mixture of methanol or acetonitrile and water and analyzed immediately by reverse-phase HPLC (HP1100) or LC-MS (Perkin Elmer API-150). All P450-catalyzed reactions were assayed according to the previous reports (Table 1). To assess the contribution of CYP2C9 to the metabolism of flurbiprofen in human liver microsomes, inhibition assays were performed in the absence and presence of the MAbs.

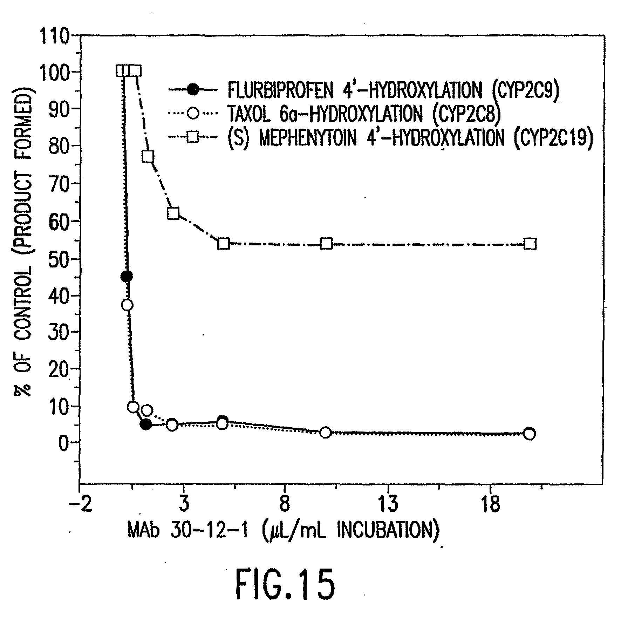

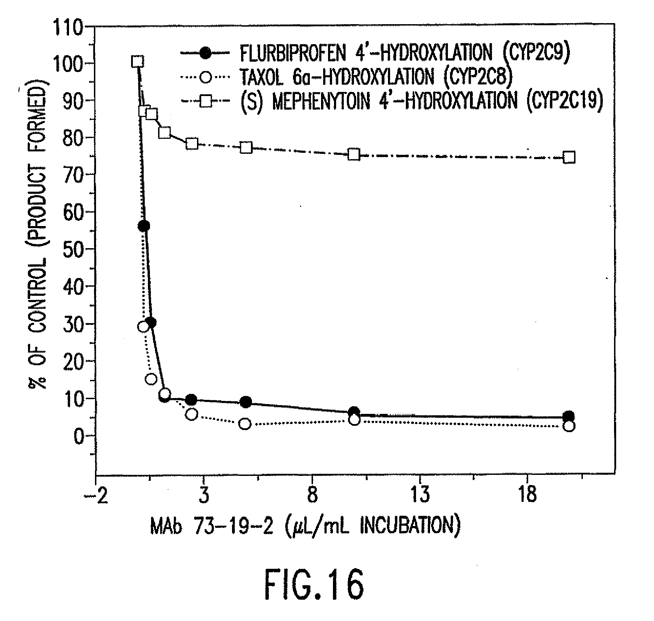

Two Mabs (30-12-1 and 73-19-2) were examined for inhibitory effect on CYP2C9-catalyzed flurbiprofen 4'-hydrozylation, CYP2C8-catalyzed taxol 6α-hydroxylation and CYP2C19-mediated mephenytoin 4'-hydroxylation. Figures 15 and 16 show the titration curve of the MAb-mediated inhibition of metabolism versus amount of mouse ascites. Mabs were found to be very inhibitory to both CYP2C8/9-mediated metabolism. Addition of 10 µl ascites of MAb30-12-1 and MAb73-19-2 to one mL incubation of substrate containing 25-50 pmol of P450, inhibited CYP2C9-catalyzed flurbiprofen 4'-hydroxylation by 96% and 97%, inhibited CYP2C8-catalyzed taxol 6α-hydroxylation by 95 and 97%, and partially inhibited CYP2C19-mediated mephenytoin 4'-hydroxylation by 46% and 24%, respectively.

### EXAMPLE 19

### HPLC Assays for Mabs 2C9

The HPLC assays are done according to the description within Example 9 except for the following addition. The product of phenacetin-O-deethylation (CYP1A2) was separated on the ODS C18 20/20 column (5µ, 4.6mm x 15 cm) eluted with 10% acetonitrile for a 6-min isocratic and then to a 9-min gradient to 60%. The metabolite (acetominophen) was detected at 250 nm and quantitated according to the ratio of chromatographic area of the metabolite to internal standard (acetanilide).

### EXAMPLE 20

### Results for Mabs 2C9

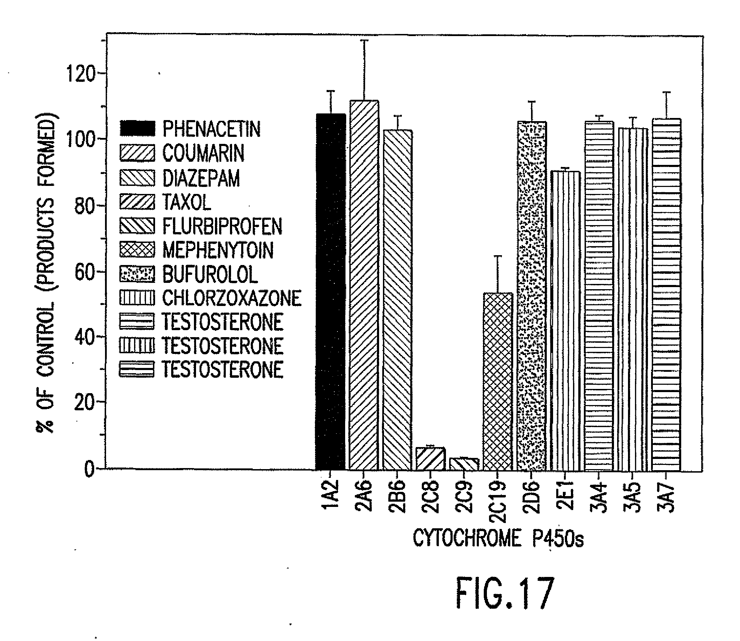

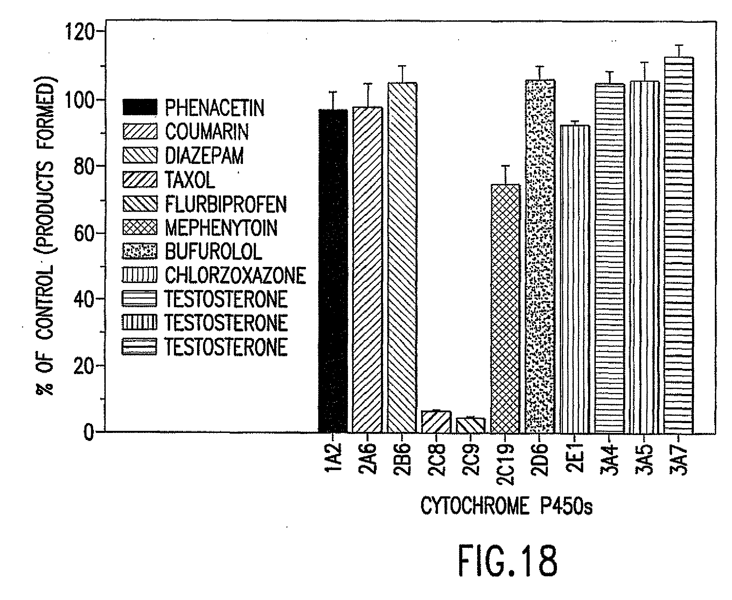

Cross-inhibition with other human P450s: To confirm the specificity of the MAbs, assays for individual cDNA-expressed P450s were performed as shown in Figs. 17 and 18. Ten µL of MAb30-12-1 or MAb73-19-2 ascites were added separately to one mL of incubation of substrate with individual P450s (15-30 pmol per mL incubation) to examine cross-inhibition. Results show that the two MAbs inhibited strongly CYP2C8/9-catalyzed reaction, and partially cross-inhibited CYP2C19 activity, but did not significantly display any inhibitory effects toward cDNA-expressed human P450-mediated phenacetin O-deethylation (CYP1A2), coumarin 7-hydroxylation (CYP2A6), diazepam *N*-demethylation (CYP2B6), bufuralol 1'-hydroxylation (CYP2D6), chlorzoxazone 6-hydroxylation (CYP2E1) and testosterone 6β-hydroxylation (CYP3A4/5/7), respectively.

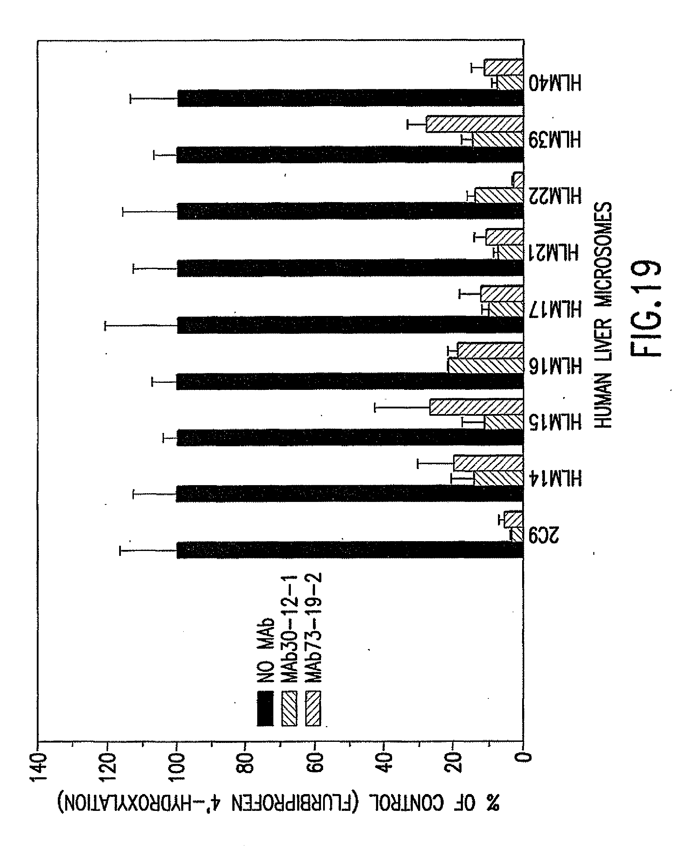

Quantitative assessment of CYP2C9 contribution to the metabolism of flurbiprofen in human liver microsomes: The selectivity of the two MAbs toward CYP2C9 also was evaluated in human liver microsomal preparations (Fig. 19). The results showed that the two MAbs inhibited almost exclusively flurbiprofen 4'-hydroxylation (73∼97%), suggesting that the 4'-position of the molecule is a major site catalyzed only by CYP2C9 isoform present in human liver. Thus, when a reaction is suspected to be catalyzed by multiple P450s in a tissue preparation, the percentage inhibition of that reaction is a reflection of contribution of CYP2C8/9 to the metabolism.

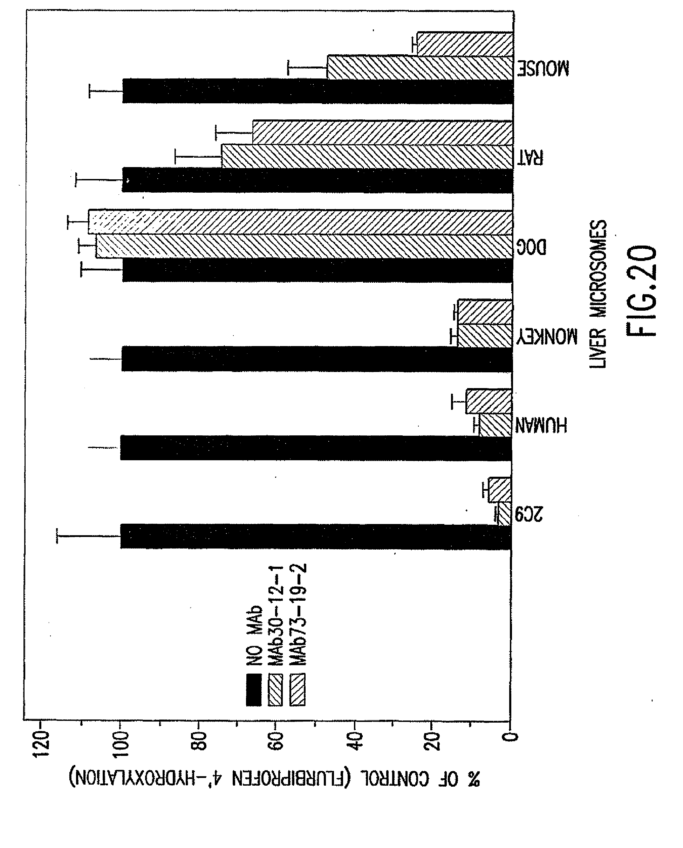

Cross-inhibition in different species: As shown in Fig. 20, the two MAbs inhibited almost completely flurbiprofen 4'-hydroxylase activity (94-98%) in liver microsomes of human and Rhesus monkey, and partially inhibited flurbiprofen 4'-hydroxylation in mouse (52 and 76%). No significant inhibition, however, was observed for rat and dog liver microsomes.

## Claims

1. A method of determining expression levels of cytochrome P450 2C9 in a sample which comprises:
(a) contacting the sample with antibody ATCC PTA-568 or ATCC PTA-569 having a detectable label; and
(b) measuring the amount of label; said amount indicating the level of expression of CYP2C9 in the sample.

2. A method of determining expression levels of cytochrome P450 2C8 in a sample which comprises:
(a) contacting the sample with antibody ATCC PTA-568 or ATC PTA-569 having a detectable label; and
(b) measuring the amount of label; said amount indicating the level of expression of CYP2C8 in the sample.

3. A method of determining expression levels of cytochrome P450 2C9 in a sample which comprises:
(a) separating a sample on a gel;
(b) transferring the separated sample to a piece of material capable of adsorbing said sample;
(c) contacting the separated sample on the material with a first antibody which is ATCC PTA-568 or ATCC PTA-569;
(d) contacting the material with a second antibody specific for the first antibody which comprises a detectable label allowing for the quantitation of the bound first antibody; and
(e) quantitating the level of antibody, same indicating the level of expression of CYP2C9 in the sample.

4. A method of determining expression levels of cytochrome P450 2C8 in a sample which comprises:
(a) separating a sample on a gel;
(b) transferring the separated sample to a piece of material capable of adsorbing said sample;
(c) contacting the separated sample on the material with a first antibody ATCC PTA-568 or ATCC PTA-569;
(d) contacting the material with a second antibody specific for the first antibody which comprises a detectable label allowing for the quantitation of the bound first antibody; and
(e) quantitating the level of antibody, same indicating the level of expression of CYP2C8 in the sample.

5. A method of determining expression levels of cytochrome P450 2C9 in a sample in accordance with claim 3 wherein:
(a) the gel is an SDS polyacrylamide gel;
(b) the adsorbing material is nitrocellulose paper;
(c) the second antibody is an alkaline phosphatase-conjugated anti-mouse IgM (µ chain); and
(d) the quantitating is carried out by treating the alkaline phosphatase-conjugated anti-mouse IgM (µ chain) on the absorbed material with a substrate for the alkaline phosphatase, metabolism of the substrate being identified by a colored product which is to be quantitated.

6. A method of determining expression levels of cytochrome P450 2C8 in a sample in accordance with claim 4 wherein:
(a) the gel is an SDS polyacrylamide gel;
(b) the adsorbing material is nitrocellulose paper;
(c) the second antibody is an alkaline phosphatase-conjugated anti-mouse IgM (µ chain); and
(d) the quantitating is carried out by treating the alkaline phosphatase-conjugated anti-mouse IgM (µ chain) on the absorbed material with a substrate for the alkaline phosphatase, metabolism of the substrate being identified by a colored product which is to be quantitated.

7. A method of identifying CYP2C9-catalyzed metabolism of a particular substrate, which comprises:
(a) contacting a sample comprising CYP2C9 and antibody ATCC PTA-568 or ATCC PTA-569 with the substrate;
(b) measuring both substrate and product levels; and
(c) comparing the substrate and product levels with levels obtained from a control sample comprising CYP2C9 and the substrate but not the antibody; a lower level of substrate-to-product conversion in the sample comprising the antibody indicating the presence of CYP2C9-catalyzed metabolism.

8. A method of identifying CYP2C8-catalyzed metabolism of a particular substrate, which comprises:
(a) contacting a sample comprising CYP2C8 and antibody ATCC PTA-568 or ATCC PTA-569 with the substrate;
(b) measuring both substrate and product levels; and
(c) comparing the substrate and product levels with levels obtained from a control sample comprising CYP2C8 and the substrate but not the antibody; a lower level of substrate-to-product conversion in the sample comprising the antibody indicating the presence of CYP2C8-catalyzed metabolism.

9. A method of quantifying CYP2C9-specific metabolism of a substrate in a sample which comprises:
(a) contacting the sample comprising both antibody ATCC PTA-568 or ATCC PTA-569 and cytochrome P450s known to be involved in the metabolism of the substrate with the substrate;
(b) measuring substrate and product levels;
(c) comparing the substrate and product levels with levels obtained from a control sample comprising both CYP2C9 and the substrate but not the antibody.

10. A method of quantifying CYP2C8-specific metabolism of a substrate in a sample which comprises:
(a) contacting the sample comprising both antibody ATCC PTA-568 or ATCC PTA-569 and cytochrome P450s known to be involved in the metabolism of the substrate with the substrate;
(b) measuring substrate and product levels;
(c) comparing the substrate and product levels with levels obtained from a control sample comprising both CYP2C8 and the substrate but not the antibody.

11. A monoclonal antibody which is ATCC PTA-568.

12. A monoclonal antibody which is ATCC PTA-569.
